# EUROPÄISCHE PATENTSCHRIFT

(11) **EP 3 578 125 B1**
(45) Veröffentlichungstag und Bekanntmachung des Hinweises auf die Patenterteilung: **08.05.2024**
(21) Anmeldenummer: 18176834.2
(22) Anmeldetag: 08.06.2018
(51) Int. Cl.: A61B 18/14, A61B 17/29, A61B 90/00

(54) **LAPAROSKOPISCHES ZANGENINSTRUMENT**
LAPAROSCOPIC FORCEPS INSTRUMENT
INSTRUMENT À PINCE LAPAROSCOPIQUE

(43) Veröffentlichungstag der Anmeldung: 11.12.2019
(73) Patentinhaber: Erbe Elektromedizin GmbH, 72072 Tübingen (DE)
(72) Erfinder: Brodbeck, Achim, 72555 Metzingen (DE); Closs, Tim, 82335 Berg (DE); Voigtländer, Matthias, 72810 Gomaringen (DE); Nold, Bernhard, 72070 Tübingen (DE)
(74) Vertreter: Rüger Abel Patentanwälte PartGmbB

(56) Entgegenhaltungen:
- WO-A1-2018/039412
- US-A1- 2010 179 545
- US-A1- 2018 132 926

## Beschreibung

Die Erfindung betrifft ein Zangeninstrument, insbesondere ein Zangeninstrument zur Gewebefusion, insbesondere ein Instrument mit verminderter Quetschneigung.

Zangenartige Instrumente zum Abklemmen, d.h. temporären Schließen von Geweben, insbesondere aber zum dauerhaften Verschließen von Gefäßen durch Fassen, Zusammendrücken und Koagulieren der Gefäße durch Bestromung sind aus dem Stand der Technik prinzipiell bekannt. Solche Instrumente weisen typischerweise zwei Branchen auf, von denen mindestens eine an einem Gelenk gegen die andere beweglich gelagert ist. Eine Schwenkbewegung der Branche führt an dem distalen Enden der Branchen zu einer relativ großen Abstandsänderung, während in Gelenknähe lediglich eine geringe Abstandsänderung vorhanden ist. Ein zwischen den Branchen gefasstes Gefäß wird deswegen entlang der Branche unterschiedlich stark zusammengedrückt. In Gelenknähe kann Quetschneigung bestehen, so dass ein Gefäß vor dem ordnungsgemäßen Versiegeln zerquetscht oder durch übermäßigen Druck anderweitig mechanisch geschädigt wird, womit ein ordnungsgemäßer Gefäßverschluss nicht mehr sichergestellt werden kann.

Dazu beschreibt die US 2016/0157923 A1 ein für den offenchirurgischen Einsatz konzipiertes Instrument in Gestalt einer Gewebefusionszange mit zwei gegeneinander beweglichen Branchen sowie mit einem Messer, mit dem ein versiegeltes Gefäß durchtrennt werden kann. Zur Vermeidung ungleichmäßigen Zusammendrückens des Gefäßes ist in Gelenknähe ein Gewebeanschlag angeordnet, der verhindert, dass Gewebe dem Gelenk zu nahe kommt.

Der Einsatz eines solchen Gewebeanschlags verhindert zwar das Vordringen des Gewebes zu dem Gelenk, wobei jedoch Gewebequetschungen weiterhin nicht ausgeschlossen sind.

Aus der WO 2018/039412 A1 ist eine Ultraschallzange mit mindestens einer beweglichen Branche bekannt. Den beiden Branchen ist ein beweglicher Gewebeanschlag zugeordnet, der beim Schließen der Branchen Raum für das gefasste Gewebe freigibt. Die Bewegung des Gewebeanschlags wird aus der Bewegung der Branchen abgeleitet.

Davon ausgehend ist es Aufgabe der Erfindung, ein verbessertes Zangeninstrument zu schaffen.

Diese Aufgabe wird mit dem medizinischen Zangeninstrument nach Anspruch 1 gelöst:

Das erfindungsgemäße Zangeninstrument weist zwei Branchen auf, von denen wenigstens eine schwenkbeweglich gelagert ist, um die Branchen aufeinander zu und voneinander weg bewegen, d.h. das Zangeninstrument öffnen und schließen zu können. Zwischen den Branchen können verschiedene Arten biologischen Gewebes, insbesondere Hohlgefäße gefasst werden, die temporär oder dauerhaft zu verschließen sind. Solche Gefäße werden zwischen den beiden Branchen gefasst und zusammengedrückt. Der an dem Instrument erfindungsgemäß vorgesehene bewegliche Gewebeanschlag verhindert dabei bei offener Zange, d.h. voneinander entfernten Branchen, zunächst, dass das gefasste Gefäß oder sonstiges biologisches Gewebe zu nah an das Gelenk des Zangeninstruments heran gelangen kann.

Der Gewebeanschlag ist beweglich angeordnet, so dass er beim Schließen des Zangeninstruments Raum für von den Branchen verdrängtes Gewebe freigeben kann. Der Gewebeanschlag ist dabei derart gelagert oder geführt, dass seine Gewebeanlagefläche beim Schließen der Branchen in Richtung auf das Gelenk hin bewegt wird oder ausweichen kann.

Die Bewegung des Gewebeanschlags kann eine geführte Bewegung sein, die an die Bewegung der wenigstens einen schwenkbeweglichen Branche gekoppelt ist. Es ist jedoch auch möglich, den Gewebeanschlag von einer Bewegung der Branchen unabhängig zu bewegen. Dazu kann der Gewebeanschlag, beispielsweise bei geöffneten Branchen, in einer ersten Position gehalten und in dieser arretiert sein, wobei die Arretierung durch Betätigung eines gesonderten Entriegelungsmittels oder infolge einer Bewegung der Branchen beim Schließen derselben lösbar ist. Nach dem Lösen der Arretierung kann der Gewebeanschlag frei beweglich oder gegen eine Federkraft beweglich sein, so dass es von dem verdrängten Gewebe bewegbar ist und diesem ausweichen kann. Der Gewebeanschlag kann auch selbst als Feder ausgebildet sein.

Bei einer bevorzugten Ausführungsform des Zangeninstruments sind beide Branchen aufeinander zu und voneinander weg beweglich gelagert. Damit lässt sich auf einfache Weise ein großer Öffnungswinkel des Zangeninstruments erreichen, so dass große Gewebevolumina und großvolumige Gefäße gefasst werden können. Im Zusammenhang mit diesem Merkmal bietet der bewegliche gelagerte Gewebeanschlag einen guten Quetschschutz für gefasstes Gewebe. Insbesondere bei Instrumenten, bei denen beide Branchen um die gleiche Gelenkachse schwenkbar gelagert sind, wird so eine Quetsch- oder Einklemmneigung des gefassten Gewebes oder von Gefäßen im Gelenkbereich drastisch reduziert. Somit wird das Risiko einer mechanischen Gewebezerstörung vermindert oder vollständig gebannt. Ist das Zangenelement beispielsweise eine Klemme zum lediglich temporären Verschließen von Gefäßen wird eine Gefäßbeschädigung vermieden. Ist das Zangeninstrument ein Instrument für den dauerhaften Gefäßverschluss, beispielsweise durch Gewebefusion durch Koagulation, wird durch die Verhinderung von Quetschungen des Gefäßes im gelenknahen Bereich eine Reduktion der Versiegelungsfestigkeit oder gar das Versagen der Versieglung verhindert oder wenigstens die Neigung dazu gemindert. Auch wird eine ungewollte Durchtrennung des Gefäßes unterbunden.

Mit der Erfindung wird ein ausreichender Abstand zwischen gegriffenem Gewebe und dem Gelenk des Zangeninstruments sichergestellt. Dieser ausreichende Abstand ist für verschiedene Mengen gefassten Gewebes und für verschiedene Gefäßdurchmesser unterschiedlich. Durch eine Bewegung des Gewebeanschlags in Abhängigkeit vom Öffnungswinkel des Werkzeugs, ist der jeweils für die gegriffene Gewebemenge bzw. die Größe des gegriffenen Gefäßes passende Sicherheitsabstand zwischen dem Gewebe und dem Gelenk sicher garantiert.

Mit der Erfindung lassen sich Quetschzonen am biologischen Gewebe im gelenknahen Bereich verringern oder vermeiden. Durch die Minimierung des Risikos des Einklemmens vom Gewebe im Gelenkbereich wird die Funktion des Zangeninstruments sicherer und insgesamt verbessert. Auch wird eine Gewebeschädigung durch zu hohe Presskräfte, wie sie sonst im gelenknahen Bereich auftreten könnten, vermieden und gegebenenfalls sicher ausgeschlossen.

Der Gewebeanschlag ist als Schieberelement ausgebildet und translatorisch, insbesondere linear beweglich gelagert.

Der Gewebeanschlag kann als Schieberelement ausgebildet sein, das eine ebene, einfach gekrümmte oder doppelt gekrümmte Gewebeanlagefläche aufweist, die z.B. durch ein stumpfes distales Ende des Schieberelements gebildet ist. Unter einer stumpfen Fläche wird dabei jede Fläche verstanden, die eben oder gerundet ausgebildet ist und keine spitzen, sich in das Gewebe einschneidenden scharfen Ecken oder Kanten aufweist oder von solchen begrenzt ist. Insbesondere kann die Gewebeanlagefläche eine längliche Fläche mit zylindrischer Wölbung im Mittelbereich und zusätzlicher Abrundung in den beiden seitlichen Endbereichen sein. Die Gewebeanlagefläche ist dann aus einer einfach gekrümmten Fläche in der Mitte und zwei doppelt gekrümmten Flächen an den Enden zusammengesetzt. Die Gewebeanlagefläche kann eine längliche sich im Wesentlichen parallel zur Gelenkachse erstreckende Fläche sein, die an einem Endstück des Schieberelements ausgebildet ist. Vorzugsweise ist die parallel zur Gelenkachse zu messende Breite kleiner oder höchstens etwa so groß wie die in gleicher Richtung zu messende Breite einer Branche, insbesondere der Gewebeauflagefläche der Branche.

Der Gewebeanschlag ist mit der wenigstens einen beweglich gelagerten Branche antriebsmäßig verbunden. Zur Verbindung kann eine zwischen dem Gewebeanschlag und der oder den beweglichen Branche(n) wirksame Getriebeeinrichtung dienen. Insbesondere ist der Gewebeanschlag mit einer Betätigungseinrichtung verbunden, die zur Betätigung der Branchen, insbesondere zum Schließen derselben dient. Der Gewebeanschlag ist Teil der Betätigungseinrichtung und als Schieberelement mit einem sich über das Gelenk in distaler Richtung hinaus erstreckenden Fortsatz ausgebildet. Ebenfalls ist die Getriebeverbindung zwischen den Branchen und dem Gewebeanschlag vorzugsweise derart ausgebildet, dass sich der Gewebeanschlag in Richtung auf das Gelenk hin bewegt, wenn die Branche eine Schließbewegung ausführt und so für das sich beim Schließvorgang deformierende und auch in Richtung des Gelenks ausdehnende Gewebe zusätzlichen Raum freigibt.

Ist das Zangeninstrument zum dauerhaften Gefäßverschluss vorgesehen, sind die Gewebeauflageflächen der beiden Branchen vorzugsweise als Elektroden ausgebildet und mit einer entsprechenden elektrischen Quelle, insbesondere einem HF-Generator, verbindbar. Dadurch kann das zwischen den Branchen gefasste Gewebe durch Stromdurchfluss erhitzt und koaguliert werden. Gefasste zwischen den Branchen zusammengedrückte Gefäße können fusioniert werden. Die Elektroden können über einen vom Behandler zu betätigenden Schalter mit der Stromquelle verbunden sein, so dass die Koagulation nur bedarfsweise und unter der Kontrolle des Behandlers erfolgt.

Es ist möglich, an dem erfindungsgemäßen Zangeninstrument Schneidvorrichtungen, insbesondere in Gestalt eines beweglichen mechanisch schneidenden Messers oder in Gestalt eines wenig oder nicht beweglichen durch Spannungsbeaufschlagung elektrisch schneidenden Messers vorzugsehen, die gefasstes und koaguliertes Gewebe im Versiegelungsbereich durchtrennen. Bei solchen Instrumenten verhindert der Gewebeanschlag mechanische Schädigungen des zu verschließenden Gefäßes und somit eine Beeinträchtigung der Versiegelung.

Weitere Einzelheiten vorteilhafter Ausführungsformen der Erfindung sind Gegenstand der Zeichnung, der Beschreibung oder von Ansprüchen. In der Zeichnung sind Ausführungsbeispiele der Erfindung veranschaulicht. Es zeigen:
Figur 1 ein erfindungsgemäßes Zangeninstrument für den laparoskopischen Einsatz, in perspektivischer Prinzipdarstellung,
Figur 2 das an dem distalen Ende des Instruments nach Figur 1 vorgesehene Zangenwerkzeug, in Seitenansicht,
Figur 3 das Zangenwerkzeug nach Figur 2, in perspektivischer Ansicht,
Figur 4 das Zangenwerkzeug nach den Figuren 2 und 3 mit halb geschlossenen Branchen, in Seitenansicht,
Figur 5 das Zangenwerkzeug nach Figur 4, in perspektivischer Ansicht,
Figur 6 das Zangenwerkzeug nach Figur 2 bis 5 in geschlossenem Zustand, in Seitenansicht,
Figur 7 das Zangenwerkzeug nach Figur 6, in perspektivischer Darstellung,
Figur 8 die Branchen, das Schieberelement und die zugehörige Betätigungseinrichtung des Zangeninstruments nach Figur 1 bis 7, in Seitenansicht,
Figur 9 eine Ausführungsform eines Schieberelements des erfindungsgemäßen Zangeninstruments, in perspektivischer Darstellung,
Figur 10 eine weitere Ausführungsform des Schieberelements des erfindungsgemäßen Zangeninstruments, in perspektivischer Darstellung.

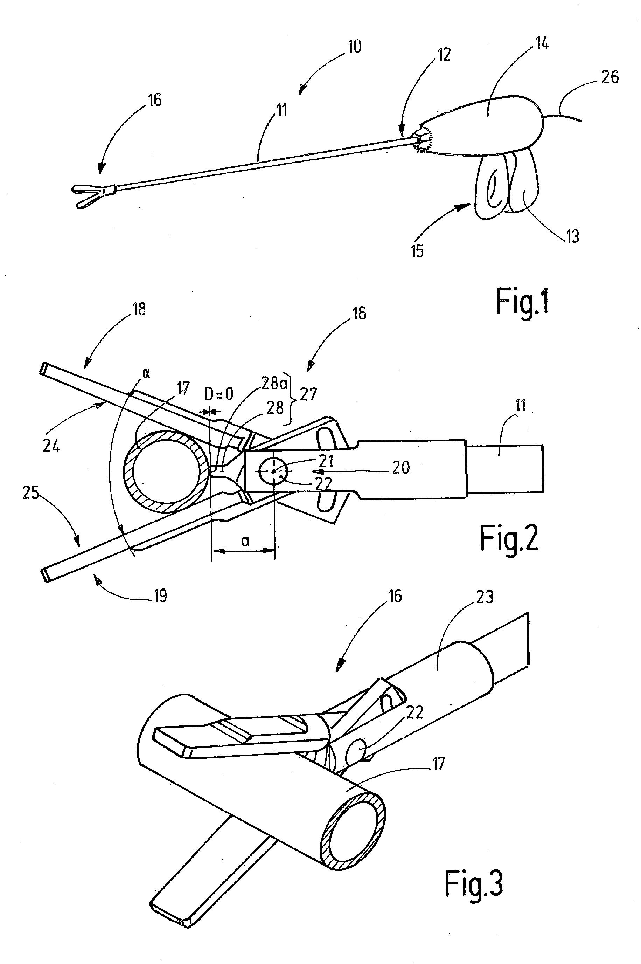

In Figur 1 ist ein Zangeninstrument 10 veranschaulicht, das für den laparaskopischen Einsatz hergerichtet ist. Es dient der Veranschaulichung der Erfindung, die gleichermaßen an Instrumenten für den offenchirurgischen Einsatz, wie auch an Instrumenten für den endoskopischen Einsatz, verwirklicht werden kann.

Das Zangeninstrument 10 nach Figur 1 weist einen länglichen steifen Schaft 11 auf, dessen proximales Ende 12 an einem mit einem Handgriff 13 versehenen Gehäuses 14 gehalten ist. In der Nähe des Handgriffs 13 ist ein Betätigungselement 15, beispielsweise in Gestalt eines Schwenkhebels, angebracht.

An dem distalen Ende des Schafts 11 ist ein zangenartiges Werkzeug 16 gehalten, dessen Aufbau und Funktion aus den Figuren 2 und 3 besser hervorgeht und das zum Fassen und/oder Klemmen von Gewebe insbesondere biologischem Gefäßen wie zum Beispiel den in den Figuren 2 und 3 dargestellten Gefäß 17 dient. Zum Öffnen und Schließen des Werkzeugs ist das Betätigungselement 15 vorgesehen, das dazu antriebsmäßig mit dem Werkzeug verbunden ist.

Zu dem Werkzeug 16 gehören zwei Branchen 18, 19, von denen wenigstens eine oder, wie es die Figuren 2 und 3 veranschaulichen, beide schwenkbar gelagert sind. Zur Lagerung dient ein Gelenk 20, das eine quer zu dem Schaft 11 gerichtete Gelenkachse 21 festlegt, um die die beiden Branchen 18, 19 schwenkbar gelagert sind. Dazu ist ein Gelenkbolzen 22 in einem gegabelten Gelenkträger 23 an beiden Enden gefasst, wobei die Branchen 18, 19 in seinem mittleren Bereich auf diesem sitzen.

Die Branchen 18, 19 weisen jeweils ebene oder auch ein-, zwei- oder dreidimensional profilierte Gewebeauflageflächen 24, 25 auf, zwischen denen das Gefäß 17 oder sonstiges biologisches Gewebe zu fassen ist. Die Gewebeauflagenflächen 24, 25 können als ebene, einfach gekrümmt oder doppelt gekrümmte, gewellte, gezahnte Flächen und als voneinander isolierte Elektroden ausgebildet sein, die über entsprechende elektrische Leiter und ein Kabel 26 (Figur 1) mit einem nicht weiter veranschaulichten speisenden Generator verbunden sind. Der Generator und die Elektroden dienen dazu, zwischen den Gewebeauflageflächen 24, 25 gefasstes, zusammengedrücktes biologisches Material elektrisch zu durchströmen und dadurch zur Bewirkung eines gewünschten chirurgischen Effekts zu erhitzen.

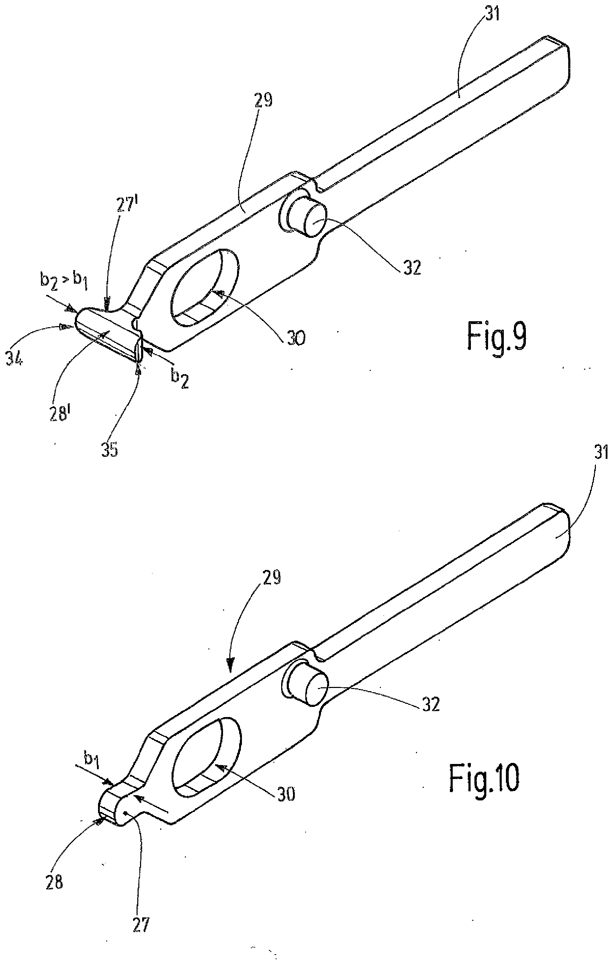

Das Werkzeug 16 weist als Gewebeanschlag 27 ein Schieberelement 28 auf, dessen gerundetes distales Ende eine Gewebeanlagefläche 28a festlegt. Zur ergänzenden Veranschaulichung wird auf Figur 10 verwiesen, in der das Schieberelement 28 mit seiner gerundeten Gewebeanlagefläche 28a in einer Ausführungsform gesondert veranschaulicht ist. Es ist dabei als distaler Fortsatz eines Getriebeelements 29 ausgebildet, das eine Langlochaussparung 30 aufweist. Der Fortsatz weist eine Breite b1 auf, die z.B. mit der Breite des Getriebeelements 29 übereinstimmt und die geringer ist, als die in gleicher Richtung parallel zu der Gelenkachse 21 zu messende Breite der Auflagefläche 24, 25.

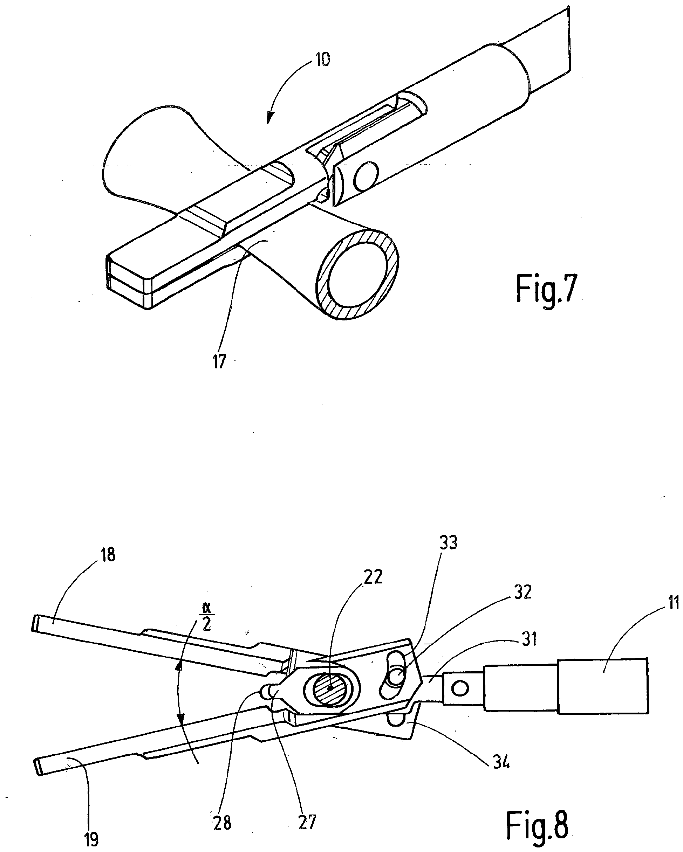

In montiertem Zustand erstreckt sich der Gelenkbolzen 22, wie Figur 8 zeigt, durch diese Langlochaussparung 30. Die ovale Langlochaussparung 30 weist in Schaftlängsrichtung vorzugsweise eine Länge auf, die zusammen mit dem Gelenkbolzen 22 ein axiales Längsspiel festlegt, das wenigstens so groß ist wie der gewünschte Axialweg des Schieberelements 28. In quer zu dem Gelenkbolzen 22 sowie quer zu der Schaftlängsrichtung zu messender Vertikalrichtung sitzt das Getriebeelement 29 jedoch im Wesentlichen spielfrei auf dem Gelenkbolzen 22.

An das Getriebeelement 29 schließt sich in proximaler Richtung ein Betätigungselement 31 an, das sich durch den Schaft 11 hindurch erstreckt oder das mit einem sich durch den Schaft 11 hindurch erstreckenden Zugelement verbunden ist. In dem Gehäuse 14 ist dieses Zugelement oder das Betätigungselement 31 selbst mit einem Betätigungsmechanismus verbunden, um eine Schwenkbewegung des Betätigungselements 15 zu dem Handgriff 13 hin in eine in proximaler Richtung gerichtete Bewegung des Getriebeelements 29 umzusetzen.

Das Getriebeelement 29 weist mindestens einen quer von dem ansonsten plattenförmigen Getriebeelement 29 weg ragenden Mitnehmerzapfen 32 auf, der zum Bewegen der Branche 19 dient. Diese weist eine Lageröffnung auf, durch die sich der Gelenkbolzen 22 mit geringem Spiel hindurch erstreckt. Dadurch ist die Branche 19 schwenkbeweglich an dem Gelenkbolzen 22 gelagert. In dem sich von dem Gelenkbolzen 22 aus gesehen in proximale Richtung erstreckenden Teil der Branche 19 ist ein gekrümmtes Langloch 33 ausgebildet, das mit dem Mitnehmerzapfen 32 zusammen eine Kulissenführung bildet wie es insbesondere in Figur 8 veranschaulicht ist.

Sind beide Branchen 18, 19 schwenkbeweglich, weist das Getriebeelement 29 an seiner Gegenseite einen eben solchen Mitnehmerzapfen auf, der aus den Figuren 9 und 10 jedoch auf der dem Betrachter abgewandten Seite des plattenförmigen Teils des Getriebeelements 29 angeordnet ist. Auch diesem in den Figuren 9 und 10 nicht sichtbaren Mitnehmerzapfen ist ein gekrümmtes Langloch 34 zugeordnet, das in dem proximalen Ende der Branche 18 angeordnet ist und mit dem betreffenden Mitnehmerzapfen eine Kulissenführung bildet.

Die Radiusvariation der Langlöcher 33, 34 ist so festgelegt, dass eine Zugbewegung des Betätigungselements 31 in proximaler Richtung zu einem Schließen der Branchen 18, 19, d.h. zu einer Bewegung aufeinander zu führt.

Das Schieberelement 28 kann, wie es Figur 9 zeigt, auch eine Breite b2 aufweisen, die größer als die Breite des Getriebeelements 29 ist. Das Schieberelement 28 bildet in diesem Fall einen Hammerkopf, der zu beiden Seiten symmetrisch über den plattenförmigen Abschnitt des Getriebeelements 29 vorsteht. Vorzugsweise ist die Breite b2, die quer zur Längsrichtung des Schafts 11 und parallel zur Gelenkachse 21 zu messen ist, nicht größer als die Breite der Branchen 18, 19. Die Gewebeanlagefläche 28` ist wie die Gewebeablagefläche 28a gerundet und frei von scharfen Kanten. Insbesondere ist die Gewebeanlagefläche 28a' bezüglich einer parallel zu der Gelenkachse 21 liegenden Achse gerundet (zum Beispiel zylindrisch) sowie ebenfalls an den stirnseitigen Enden 34, 35 gerundet ausgebildet.

Das insoweit beschriebene Zangeninstrument 10 arbeitet wie folgt:
Das Zangeninstrument 10 wird an einem Patienten mit seinem Werkzeug 16 in der Nähe des zu fassenden Gefäßes 17 positioniert. Dazu kann beispielsweise der Schaft 11 durch einen kleinen Schnitt, zum Beispiel in der Bauchdecke, in den Bauchraum geschoben werden bis das Werkzeug 16 das biologische Gewebe an einer gewünschten Stelle fasst, d.h. beispielsweise das Gefäß 12 aufnimmt. Der steife Schaft 11 ist dabei so stabil, dass durch Manipulationen des Gehäuses 14 mit dem Griff 13 das Werkzeug 16 vor und zurück sowie auch seitlich bewegt werden kann.

Ist das Gefäß 17 gemäß Figur 2 und 3 gefasst, liegt es an den Gewebeauflageflächen 24, 25 und zugleich an der Gewebeanlagefläche 28a an. Es wird deswegen in einer Distanz a von der Gelenkachse 21 gehalten. Der Abstand D des Gewebes von der Anlagefläche 28a ist dabei zum Beispiel Null.

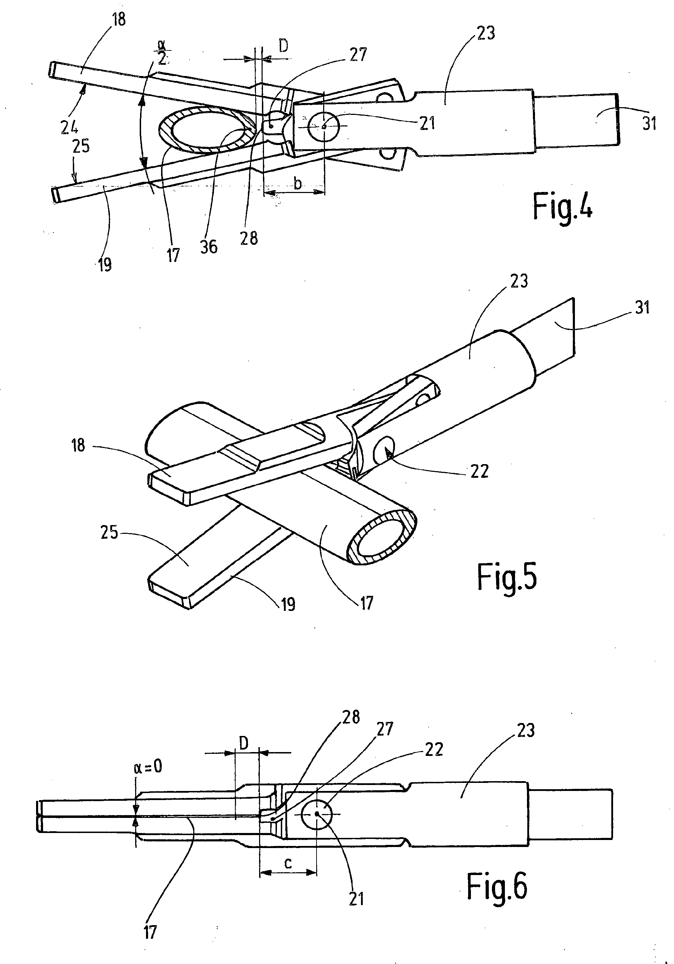

Wird nun das Zangeninstrument geschlossen, indem das Betätigungselement 31 in Schaftlängsrichtung in proximaler Richtung bewegt wird, beispielsweise durch entsprechendes Schwenken des Betätigungselements 15, wird dadurch gleichzeitig das Schieberelement 28 in proximaler Richtung bewegt. Es wird dazu auf die Figuren 4 und 5 verwiesen. Durch die Bewegung des Schieberelements 28 in proximaler Richtung verringert sich der Abstand zwischen der Gelenkachse 21 und der Gewebeanlagefläche 28a. Der sich einstellende Abstand b ist geringer als der im Zusammenhang mit Figur 2 und 3 erläuterte Abstand a bei vollständig geöffnetem Instrument.

In Figur 4 ist der Öffnungswinkel α im Vergleich zu Figur 2 halbiert. Infolgedessen ist das Gefäß 17 teilkomprimiert. Sein zuvor im Wesentlichen kreisförmiger Querschnitt wird nun zu einem Ovalquerschnitt, dessen proximale Partie 36 der Gelenkachse 21 näher liegt als in Figur 2. Weil die Rückhubbewegung des Schieberelements 28 jedoch größer ist als die Bewegung der Partie 36 des sich ausbeulenden Gefäßes ist der Abstand D des Gewebes von der Anlagefläche 28a nun größer als Null. In anderen Worten, es besteht keine Anlage mehr zwischen der Anlagefläche 28 und dem biologischen Gewebe des Gefäßes 17, so dass die proximale Ausbreitung des Gewebes nicht blockiert und der gelenknahen Anhäufung von Gewebe entgegengewirkt wird.

In Figur 6 und 7 ist das vollständig geschlossene Zangeninstrument 10 veranschaulicht (d.h. der Öffnungswinkel α ist gleich 0). Der Abstand c der Gewebeanlagefläche 28a von der Gelenkachse 21 ist minimal. Der Abstand D des Gewebes von der Anlagefläche 28a ist maximal. Dieser Abstand D bildet eine räumliche Reserve zur Ausbreitung von Gewebe. Somit können für Gefäße und Gewebearten, die stärker in proximaler Richtung ausbauchen oder auswandern und sich vornehmlich in Gelenknähe anhäufen würden Bedingungen einer gleichmäßigeren Verteilung über die Fassbereiche herbeigeführt werden was wiederum eine erfolgreiche Versiegelung der Gewebe bzw. Gefäße begünstigt.

Bei dem erfindungsgemäßen Zangeninstrument 10 ist vorzugsweise ein zentrales Zug- und Schubglied vorgesehen, das zum Beispiel durch das Getriebeelement 29 und das Betätigungselement 31 gebildet wird. Dieses zentrale Schub- und Zugglied, dient zum Öffnen und Schließen der Branchen 18, 19 wie auch als ein vom Schließgrad der Branchen 18, 19 abhängiger variabler Gewebeanschlag 27. Vorzugsweise ist der variable Gewebeanschlag 27 als Schieberelement 28 ausgebildet und linear in proximaler Richtung proportional zu dem Öffnungswinkel α der Branchen 18, 19 verstellbar. Beim Fassvorgang werden Gefäße 17 in proximaler Richtung zwischen die Branchen 18, 19 gebracht. Hierbei fungiert der Gewebeanschlag 27 als Abstandshalter zwischen dem Gelenkbereich und dem gefassten Material. Beim Schließen der Branchen 18, 19 wird das Gefäß 17 ortsfest gehalten und zusammengedrückt. Beim Zusammendrücken des Gefäßes 17 kommt es zu einer Ausdehnung desselben in Axialrichtung in distaler und proximaler Richtung. Durch Zurückweichen des variablen Gewebeanschlags 27 beim Schließvorgang wird Raum zum Ausweichen des gefassten Materials geschaffen. Gewebeanhäufungen können somit eingeschränkt und einer Überbelastung von Gewebe entgegengesetzt werden.

In einer weiteren Ausgestaltung der Erfindung ist es möglich, über die räumliche Lage des zentralen Zug- und/oder Schubelements und/oder über die Erfassung der an diesen Elementen wirkenden Kraft bzw. der benötigten Presskraft Aussagen über den Zustand der Verteilung des gefassten biologischen Materials, insbesondere des Grads der Anhäufung im gelenknahen Fassbereich, und damit der zu machen. Die Wegerfassung ist z.B. durch optisch interpretierbare Markierungen, die Krafterfassung z.B. mittels Messstreifen und/oder piezoelektrischen Materialien möglich. Die Erfassung des Grads der Gewebeverteilung über den Fassbereich und insbesondere der Gewebeanhäufung im gelenknahen Bereich kann in unterschiedlichen Betriebszuständen des Instruments erfolgen. Betriebszustände sind insbesondere der dynamische Schließvorgang und der stationäre geschlossene Zustand, während und/oder nach Abschluss der elektrochirurgischen Anwendung erfolgen.

Anhand der erfassten Daten können Anpassungen an Modi für die Bereitstellung der elektrochirurgischen Wirkeffekte an Gewebe bzw. Gefäßen über das Instrument gemacht werden. Dadurch kann sich das Instrument über einen interpretierenden Mode an wechselnde Interaktionsbedingungen mit Geweben bzw. Gefäßen unterschiedlicher mechanischer Beschaffenheit, insbesondere varianter Deformierbarkeit, Steifigkeit und Mächtigkeit, anpassen.

### Bezugszeichen:

- 10: Zangeninstrument
- 11: Schaft
- 12: proximales Ende des Schafts 11
- 13: Handgriff
- 14: Gehäuse
- 15: Betätigungselement
- 16, 16a: Werkzeug
- 17: Gefäß
- 18: erste (obere) Branche
- 19: zweite (untere) Branche
- 20: Gelenk
- 21: Gelenkachse
- 22: Gelenkbolzen
- 23: Gelenkträger
- 24: Gewebeauflagefläche der ersten Branche 18
- 25: Gewebeauflagefläche der zweiten Branche 19
- 26: Kabel
- 27`: Gewebeanschlag
- 28, 28': Schieberelement
- 28a, 28a', 28 a": Gewebeanlagefläche
- 29: Getriebeelement
- 30: Langlochaussparung
- 31: Betätigungselement
- 32: Mitnehmerzapfen
- 33: Langloch
- 34, 35: gerundete Enden des Schieberelements 28, 27'
- a, b, c: Distanz des Gewebes von der Gelenkachse 21
- D: Abstand des Gewebes von der Anlagefläche 28
- 36: proximaler Teil des Gefäßes 17
- 37: Feder

## Patentansprüche

1. Medizinisches Zangeninstrument (10), insbesondere zur Gewebefusion,
mit einem Werkzeug (16), das zwei Branchen (18, 19) zum Fassen von biologischem Gewebe zwischen einander aufweist,
mit einem Gelenk (20), an dem wenigstens eine der Branchen (18, 19) um eine Gelenkachse (21) schwenkbeweglich gelagert ist, um auf die andere Branche (18, 19) hin und von dieser weg bewegt zu werden,
mit einem Gewebeanschlag (27), der bei dem Gelenk (20) beweglich angeordnet ist und ein Schieberelement (28) aufweist, dessen gerundetes distales Ende eine Gewebeanlagefläche (28a) festlegt,
mit einem zentralen Zug- und Schubglied, das zum Öffnen und Schließen der Branchen (18, 19) dient und das durch ein Getriebeelement (29) und ein Betätigungselement (31) gebildet ist,
wobei der Gewebeanschlag (27) als distaler Fortsatz des Getriebeelements (29) ausgebildet ist.

2. Zangeninstrument nach Anspruch 1, **dadurch gekennzeichnet, dass** beide Branchen (18, 19) aufeinander zu und voneinander weg beweglich gelagert sind.

3. Zangeninstrument nach einem der vorstehenden Ansprüche, **dadurch gekennzeichnet, dass** der Gewebeanschlag (27) linear beweglich gelagert ist.

4. Zangeninstrument nach einem der vorstehenden Ansprüche, **dadurch gekennzeichnet, dass** die Gewebeanlagefläche (28a) durch ein stumpfes distales Ende des Gewebeanschlags (27) gebildet ist.

5. Zangeninstrument nach einem der vorstehenden Ansprüche, **dadurch gekennzeichnet, dass** die Gewebeanlagefläche (28a) eine parallel zu der Gelenkachse (21) zu messende Querabmessung (b1, b2) aufweist, die geringer ist, als die in gleicher Richtung zu messende Breite einer der Branchen (18, 19).

6. Zangeninstrument nach einem der vorstehenden Ansprüche, **dadurch gekennzeichnet, dass** das der Gewebeanschlag (27) als Schieberelement (28) ausgebildet ist und einen die Gewebeanlagefläche (28a) tragenden Vorsprung aufweist, dessen parallel zur Gelenkachse (21) zu messende Breite (b1, b2) größer ist als die restliche Breite des Schieberelements (27).

7. Zangeninstrument nach einem der vorstehenden Ansprüche, **dadurch gekennzeichnet, dass** die Betätigungseinrichtung (31) über eine Getriebeeinrichtung (29) mit der wenigstens einen beweglich gelagerten Branche (18, 19) verbunden ist.

8. Zangeninstrument nach einem der vorstehenden Ansprüche, **dadurch gekennzeichnet, dass** die Getriebeeinrichtung (29, 32) ein Kulissengetriebe ist.

9. Zangeninstrument nach einem der Ansprüche, 7 oder 8 **dadurch gekennzeichnet, dass** die Getriebeeinrichtung (29, 32) derart ausgebildet ist, dass der Gewebeanschlag bei einer Schließbewegung der Branchen (18, 19) eine Ausweichbewegung in Richtung auf das Gelenk (20) ausführt.

10. Zangeninstrument nach einem der vorstehenden Ansprüche, **dadurch gekennzeichnet, dass** die beiden Branchen (18, 19) mit Elektroden versehen sind oder als Elektroden ausgebildet sind, die mit einer elektrischen Spannung beaufschlagbar sind.

## Claims

1. A medical forceps instrument (10), in particular for tissue fusion, comprising:
a tool (16) that comprises two branches (18, 19) for grasping biological tissue between said branches,
a hinge (20) that supports at least one of the branches (18, 19) so as to be movable in a pivoting manner about a hinge axis (21) in order to be able to be moved toward or away from the other branch (18, 19), and
a tissue abutment (27) that is movably arranged on the hinge (20) and having a slider element (28) whose rounded distal end defines a tissue abutment surface (28a),
a central pull-push member that serves for opening and closing the branches (18, 19) and is formed by a gearing element (29) and an actuating element (31),
wherein the tissue abutment (27) is formed as a distal extension of a gearing element (29).

2. The forceps instrument according to Claim 1, **characterized in that** both branches (18, 19) are supported so as to be movable toward each other and away from each other.

3. The forceps instrument according to one of the previous claims, **characterized in that** the tissue abutment (27) is supported so as to be linearly movable.

4. The forceps instrument according to one of the previous claims, **characterized in that** the tissue abutment surface (28a) is configured as a blunt distal end of the tissue abutment (27).

5. The forceps instrument according to one of the previous claims, **characterized in that** the tissue abutment surface (28a) has a transverse dimension (b1, b2) to be measured parallel to the hinge axis (21), said transverse dimension being smaller than the width of a branch (18, 19) to be measured in the same direction.

6. The forceps instrument according to one of the previous claims, **characterized in that** the tissue abutment (27) is configured as the slider element (28) and has a tissue abutment surface (28a) bearing a projection whose width (b1, b2) to be measured parallel to the hinge axis (21) is larger than the remaining width of the slider element (27).

7. The forceps instrument according to one of the previous claims, **characterized in that** the actuating arrangement (31) is connected to the at least one movably supported branch (18, 19) via a gearing arrangement (29).

8. The forceps instrument according to one of the previous claims, **characterized in that** the gearing arrangement (29, 32) is a connecting link gear.

9. The forceps instrument according to one of the Claims 7 or 8, **characterized in that** the gearing arrangement (29, 32) is configured in such a manner that the tissue abutment performs an escape movement in the direction toward the hinge (20) during a closing movement of the branches (18, 19) .

10. The forceps instrument according to one of the previous claims, **characterized in that** the two branches (18, 19) are provided with electrodes or are configured as electrodes to which an electrical voltage can be applied.

## Revendications

1. Instrument à pince médical (10), destiné en particulier à la fusion tissulaire,
comprenant un outil (16) qui présente deux branches (18, 19) destinées à saisir entre elles des tissus biologiques,
comprenant une articulation (20) sur laquelle au moins une des branches (18, 19) est montée avec possibilité de pivotement autour d'un axe d'articulation (21) afin de pouvoir être déplacée en direction de l'autre branche (18, 19) et en être éloignée,
comprenant une butée à tissus (27) qui est disposée de façon mobile près de l'articulation (20) et présente un élément coulissant (28) dont l'extrémité distale arrondie définit une surface d'appui de tissus (28a),
comprenant un organe de traction et poussée central qui sert à l'ouverture et à la fermeture des branches (18, 19) et qui est constitué d'un élément de transmission (29) et d'un élément d'actionnement (31),
la butée à tissus (27) étant réalisée sous forme de prolongement distal de l'élément de transmission (29).

2. Instrument à pince selon la revendication 1, **caractérisé en ce que** les deux branches (18, 19) sont montées de façon mobile, de manière à pouvoir être rapprochées et éloignées l'une de l'autre.

3. Instrument à pince selon l'une des revendications précédentes, **caractérisé en ce que** la butée à tissus (27) est montée avec possibilité de déplacement linéaire.

4. Instrument à pince selon l'une des revendications précédentes, **caractérisé en ce que** la surface d'appui de tissus (28a) est formée par une extrémité distale émoussée de la butée à tissus (27).

5. Instrument à pince selon l'une des revendications précédentes, **caractérisé en ce que** la surface d'appui de tissus (28a) présente une dimension transversale (b1, b2) à mesurer parallèlement à l'axe d'articulation (21), qui est plus faible que la largeur de l'une des branches (18, 19), à mesurer dans la même direction.

6. Instrument à pince selon l'une des revendications précédentes, **caractérisé en ce que** la butée à tissus (27) est réalisée sous forme d'élément coulissant (28) et présente une saillie qui porte la surface d'appui de tissus (28a) et dont la largeur (b1, b2) à mesurer parallèlement à l'axe d'articulation (21) est plus grande que la largeur restante de l'élément coulissant (27).

7. Instrument à pince selon l'une des revendications précédentes, **caractérisé en ce que** le dispositif d'actionnement (31) est relié par l'intermédiaire d'un dispositif de transmission (29) à la branche (18, 19), au nombre d'au moins une, montée de façon mobile.

8. Instrument à pince selon l'une des revendications précédentes, **caractérisé en ce que** le dispositif de transmission (29, 32) est un mécanisme à coulisse.

9. Instrument à pince selon l'une des revendications 7 ou 8, **caractérisé en ce que** le dispositif de transmission (29, 32) est réalisé de manière à ce que lors d'un mouvement de fermeture des branches (18, 19), la butée à tissus exécute un mouvement d'évitement en direction de l'articulation (20).

10. Instrument à pince selon l'une des revendications précédentes, **caractérisé en ce que** les deux branches (18, 19) sont pourvues d'électrodes ou sont réalisées sous forme d'électrodes auxquelles peut être appliquée une tension électrique.
